(19) Europäisches Patentamt
European Patent Office
Office européen des brevets

(11) Veröffentlichungsnummer: **0 289 939**
**A1**

(12) # EUROPÄISCHE PATENTANMELDUNG

(21) Anmeldenummer: **88106802.7**

(22) Anmeldetag: **28.04.88**

(51) Int. Cl.[4]: **C07D 403/04 , C07D 403/14 , A61K 31/50**

(30) Priorität: **08.05.87 DE 3715273**

(43) Veröffentlichungstag der Anmeldung: **09.11.88 Patentblatt 88/45**

(84) Benannte Vertragsstaaten: **AT BE CH DE ES FR GB GR IT LI LU NL SE**

(71) Anmelder: **ASTA Pharma AG**
**Weismüllerstrasse 45**
**D-6000 Frankfurt am Main 1(DE)**

(72) Erfinder: **Engel, Jürgen, Dr.**
**Erlenweg 3**
**D-8755 Alzenau(DE)**

(54) **Neue 4-Benzyl-1-(2H)-phthalazinon-Derivate mit einem Aminosäurerest.**

(57) Antiasthmatisch wirkende Verbindungen der Formel

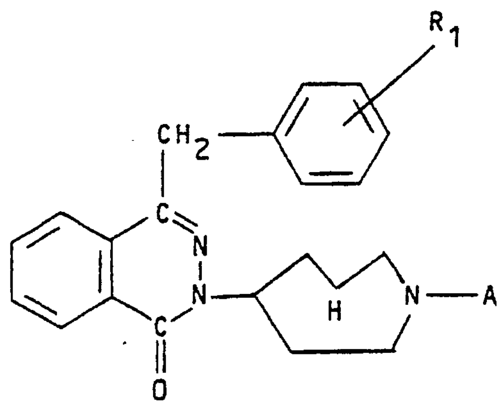

I

worin $R_1$ Wasserstoff, Fluor, Chlor, Brom, Trifluormethyl, $C_1$-$C_6$-Alkyl oder $C_1$-$C_6$-Alkoxy bedeuted, und der Rest A die Gruppe

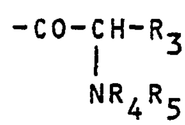

darstellt, worin $R_3$ Wasserstoff, einen Phenylrest, einen Indolyl-(3)-methylrest, Imidazolyl-(4)-methylrest, eine $C_1$-$C_{10}$-Alkylgruppe bedeutet oder worin $R_3$ eine $C_1$-$C_{10}$-Alkylgruppe bedeutet, welche durch eine Carboxygruppe, eine $C_1$-$C_6$-Alkoxycarbonylgruppe, eine Aminocarbonylgruppe, eine Hydroxygruppe, eine $C_1$-$C_6$-Alkoxygruppe, eine $C_2$-$C_6$-Alkanoyloxygruppe, eine Mercaptogruppe, eine $C_1$-$C_6$-Alkylthiogruppe, eine $C_2$-$C_6$-Alkanoylmercaptogruppe, eine Phenylgruppe, eine Hydroxyphenylgruppe, eine Dihydroxyphenylgruppe, eine Amino-$C_1$-$C_6$-alkylthiogruppe, eine Amino-$C_1$-$C_6$ alkyloxygruppe, eine Aminogruppe, eine

EP 0 289 939 A1

Xerox Copy Centre

Ureidogruppe ($H_2NCONH$-) oder eine Guanidinogruppe substituiert ist, oder worin $R_3$ zusammen mit dem Strukturteil $>CH(NHR_4)$ den Pyrrolidin-2-yl-rest (Prolinrest) oder den 4-Hydroxy-pyrrolidin-2-yl-rest darstellt, $R_4$ Wasserstoff, Benzyl oder einen $C_1$-$C_6$-Alkylrest, $R_5$ Wasserstoff, Benzyl einen $C_1$-$C_6$-Alkylrest, einen $C_2$-$C_6$-Alkanoylrest oder die Gruppe

$$-CO-\underset{NR_4R_6}{\underset{|}{CH}}-R_3$$

bedeutet, und $R_6$ Wasserstoff, Benzyl oder $C_2$-$C_6$-Alkanoyl ist, und Verfahren zu deren Herstellung.

## Neue 4-Benzyl-1-(2H)-pthtalazinon-Derivate mit einem Aminosäurerest

Gegenstand der deutschen Patentschrift 21 64 058 sind basisch substituierte 4-Benzyl-1-(2H)-phthalazinon-Derivate der folgenden Formel

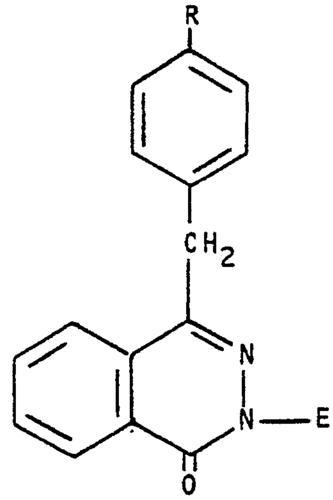

worin R ein Wasserstoff-oder Halogenatom, eine Trifluormethylgruppe oder eine niedere Alkyl-oder Alkoxygruppe und E einen 4-Perhydroazepinyl-, N-Methyl-4-perhydroazepinyl-, 3-Chinuclidyl-, 3-Tropanyl-, 3-Nortropanyl-, N-Methyl-3-pyrrolidinyl-oder N-Methyl-2-pyrrolidinyl-methyl-Rest bedeutet, sowie die physiologisch unbedenklichen Säureadditionssalze hiervon.

Diese Verbindungen besitzen eine Antihistamin-Wirkung. Demgegenüber zeichnen sich die erfindungsgemäßen Verbindungen der Formel I durch folgende überraschende Wirkung aus: Sie wirken antiasthmatisch, antiallergisch, Paf-antagonistisch (Paf = platelet activating factor, Mediator, der unter anderem Asthma auslöst) sowie Leukotrien-inhibierend.

Die folgenden Erläuterungen stellen erfindungswesentliche Angaben dar.

Der Rest $R_1$ befindet sich vorzugsweise in 4-Stellung des Phenylringes.

Die in der Formel I vorkommenden Alkylgruppen, Alkoxygruppen, Alkylaminogruppen, Alkanoylaminogruppen, Alkanoyloxygruppen, Alkanoylmercaptogruppen beziehungsweise ganz allgemein Alkanoylgruppen können gerade oder verzweigt sein. Dasselbe gilt auch für Alkyl-und Alkyloxygruppen (= Alkoxygruppen), falls diese Bestandteil anderer zusammengesetzter Gruppen sind (zum Beispiel in Form einer Monoalkyl- oder Dialkylaminogruppe, Alkanoylaminogruppe, Alkoxycarbonylgruppe, Aminoalkylthiogruppe, Aminoalkyloxygruppe und analoge Gruppen).

Bei den Halogenatomen handelt es sich insbesondere um Chlor und Fluor. Die Alkyl-und Alkoxygruppen als solche oder als Bestandteil von anderen zusammengesetzten Gruppen bestehen insbesondere aus 1-4 C-Atomen, vorzugsweise 1 oder 2 C-Atomen. Alkanoylgruppen, wie zum Beispiel Alkanoylaminogruppen, Alkanoyloxygruppen oder Alkanoylmercaptogruppen, bestehen insbesondere aus 2-4, vorzugsweise 2-3 C-Atomen.

$R_3$ ist beispielsweise eine $C_1$-$C_6$-Alkylgruppe, die in 1-, 2-, 3-, 4-, 5-oder 6-Stellung (Zählung beginnt stets an der Verknüpfungsstelle des Alkylrestes mit dem Restmolekül) eine Aminogruppe (insbesondere in 3-oder 4-Stellung), eine Mercapto-oder Hydroxygruppe (insbesondere in 1-oder 2-Stellung), eine Amino-$C_2$-$C_4$-alkylthiogruppe, eine Amino-$C_2$-$C_4$-alkoxygruppe, eine Carboxygruppe, eine $C_1$-$C_6$-Alkoxycarbonylgruppe, eine Ureidogruppe oder einen Guanidinorest enthält. Beispielsweise liegen der Gruppe -CO-CH-($NR_4R_5$)-$R_3$ beziehungsweise -CO-CH($NR_4R_6$) $R_3$ die folgenden Aminosäuren zugrunde: Asparaginsäure (DL-Form), Asparagin, $\alpha$-Amino-buttersäure, Leucin, Isoleucin, Asparaginsäureethylester (L-Form), Citrullin ($H_2N$-CO-NH-$(CH_2)_3$-CH($NH_2$)-$CO_2H$ (L-Form), Ornithin (L-Form), Arginin, 4-Thialysin ($H_2N$-$CH_2$-$CH_2$-S-$CH_2$-CH($NH_2$)-COOH), 2,6-Diamino-önanthsäure ($\epsilon$ -Methyllysin), 4-Oxalysin ($H_2N$-$CH_2CH_2$-O-$CH_2$-CH($NH_2$)-COOH), Glycin, N-Methyl-glycin, N,N-Dimethylglycin, Prolin, Hydroxyprolin, Alanin, $\beta$-Alanin, 3,4-Dihydroxyphenylalanin, Phenylalanin, Tyrosin, Tryptophan, Cystein, Homocystein (DL-Form), Methionin, Penicillamin, Lysin (insbesondere L-Lysin), Valin, Valinmethylester (L-Form), Threonin, Histidin, Serin, Homoserin, Glutaminsäure, Glutamin, $\alpha,\beta$-Diaminopropionsäure, Sarkosin, Ethionin, $\alpha,\gamma$-Diamino-buttersäure (L-Form), $\alpha$-

Aminoadipinsäure (L-Form).

Gemäß dem Herstellungsverfahren können in den Verfahrensprodukten vorhandene Hydroxy-, Mercapto-und/oder primäre beziehungsweise sekundäre Aminogruppen alkyliert oder acyliert werden. Die Alkylierung erfolgt beispielsweise durch Umsetzung mit Verbindungen der Formel MHal, $ArSO_2OM$ und $SO_2(OM)_2$, wobei Hal ein Halogenatom (insbesondere Chlor, Brom oder Jod) und Ar ein aromatischer Rest (zum Beispiel ein gegebenenfalls durch einen oder mehrere niedere Alkylreste substituierter Phenyl-oder Naphthylrest und M ein $C_1$-$C_6$-Alkylrest, ein Phenyl-$C_1$-$C_4$-alkylrest, ein Halogenphenyl-$C_1$-$C_4$-alkylrest oder ein Amino-$C_1$-$C_6$-Alkylrest mit geschützter Aminogruppe ist. Beispiele sind p-Toluolsulfonsäure-$C_1$-$C_6$-alkylester, $C_1$-$C_6$-Dialkylsulfate, $C_1$-$C_6$-Alkylhalogenide und entsprechende. Bei den zuvor genannten Verbindungen kann die Alkylgruppe gerade oder verzweigt sein. Die Alkylierungs-und Acylierungsreaktion wird gegebenenfalls unter Zusatz von üblichen säurebindenden Mitteln, wie Alkalihydroxiden, Alkalicarbonaten, Alkalihydrogencarbonaten, Erdalkalicarbonaten, Alkaliacetaten, tertiären Aminen (zum Beispiel Trialkylaminen wie Triethylamin), Pyridin oder auch Alkalihydriden bei Temperaturen zwischen 0 und 200° C, vorzugsweise 40 und 140° C in inerten Lösungsmitteln oder Suspensionsmitteln durchgeführt. Als Lösungs- oder Dispergiermittel kommen beispielsweise in Betracht: aromatische Kohlenwasserstoffe wie zum Beispiel Benzol, Toluol, Xylol; aliphatische Ketone wie zum Beispiel Aceton, Methylethylketon; halogenierte Kohlenwasserstoffe wie zum Beispiel Chloroform, Tetrachlorkohlenstoff, Chlorbenzol, Methylenchlorid; aliphatische Ether wie zum Beispiel Butylether; cyclische Ether wie zum Beispiel Tetrahydrofuran, Dioxan; Sulfoxide wie zum Beispiel Dimethylsulfoxid; tertiäre Säureamide wie zum Beispiel Dimethylformamid, N-Methyl-pyrrolidon, Hexamethylphosphorsäuretriamid; aliphatische Alkohole wie Methanol, Ethanol, Isopropanol, Amylalkohol, tert.-Butanol, cycloaliphatische Kohlenwasserstoffe wie Cyclohexan und ähnliche. Auch wässrige Mischungen der genannten Lösungsmittel können verwendet werden. Häufig arbeitet man bei der Rückflußtemperatur der verwendeten Lösungsbeziehungsweise Dispergiermittel. Häufig werden die Alkylierungsreaktionskomponenten im Überschuß eingesetzt. Die Alkylierung kann auch in Gegenwart von Tetraalkylammoniumsalzen (insbesondere der Halogenide) in Kombination mit Alkalihydroxiden bei Temperaturen zwischen 0 - 100° C, vorzugsweise 20 - 80° C in einem aprotischen Lösungsmittel oder auch in Chloroform oder Methylenchlorid vorgenommen werden. Als aprotische Lösungsmittel kommen insbesondere in Betracht: tertiäre Amide (Dimethylformamid, N-Methyl-Pyrrolidon, Hexamethylphosphorsäuretriamid), Dimethylsulfoxid, Acetonitril, Dimethoxyethan, Aceton, Tetrahydrofuran.

Bei der Acylierung wird zum Beispiel eine $C_2$-$C_6$-Alkanoylgruppe, eine $C_1$-$C_6$-Alkylcarbonylgruppe, eine gegebenenfalls durch eine oder zwei $C_1$-$C_6$-Alkylreste substituierte Carbamoylgruppe oder die Gruppe $-CO-CH(NR_4R_6)-R_3$ eingeführt. Man verfährt hierbei in an sich bekannter Weise vorzugsweise unter Verwendung der entsprechenden Halogenide (zum Beispiel Carb-$C_1$-$C_6$-alkoxyhalogeniden, $C_2$-$C_6$-Alkanoylhalogeniden), der entsprechenden Anhydride oder auch unter Verwendung der entsprechenden Säuren in Gegenwart von bekannten Kondensationsmitteln (siehe zum Beispiel Verfahren a)). Die Reaktionstemperaturen liegen beispielsweise zwischen 30 und 120° C.

Gegebenenfalls kann man bei der Alkylierung und der Acylierung auch so vorgehen, daß man zuerst von der zu alkylierenden beziehungsweise acylierenden Verbindung eine Alkaliverbindung (Natrium-, Kalium-oder auch Lithiumsalz zum Beispiel) herstellt, indem man sie in einem inerten Lösungsmittel wie Dioxan, Dimethylformamid, Benzol oder Toluol mit einem Alkalimetall, Alkalihydrid oder Alkaliamiden (insbesondere Natrium oder Natriumverbindungen) oder Butyllithium bei Temperaturen zwischen 0 und 150° C umsetzt und dann das alkylierende Agens zufügt.

Anstelle der angeführten Alkylierungs-und Acylierungsmittel können auch andere in der Chemie gebräuchliche chemischäquivalente Mittel verwendet werden (siehe zum Beispiel auch L.F. und Mary Fieser "Reagents for Organic Synthesis", John Wiley and Sons, Inc., New York, 1967, Vol. 1, Seiten 1303-4 und Vol. 2, Seite 471).

In den verwendeten Ausgangsstoffen für das erfindungsgemäße Verfahren können vorhandene Hydroxygruppen, Mercaptogruppen, Carboxygruppen, Aminogruppen oder $C_1$-$C_6$-Alkylaminogruppen vorkommen, die durch übliche Schutzgruppen geschützt sind.

Es handelt sich hierbei um übliche Schutzgruppen, die durch Hydrolyse oder Hydrogenolyse leicht abspaltbar sind und während oder nach der Reaktion abgespalten werden. Es handelt sich hierbei um Schutzgruppen, die zum Beispiel in dem Buch von J.F.W. McOmie, Protective Groups in Organic Chemistry, Plenum Press, New York, 1973, Seiten 43 - 143 sowie 183 - 215 angegeben sind.

Falls solche Schutzgruppen bei der Verfahrensreaktion nicht abgespalten werden, dann erfolgt eine Abspaltung nach der Reaktion. Häufig enthalten die Ausgangsverbindungen aufgrund ihrer Herstellung bereits derartige Schutzgruppen.

Bei diesen Schutzgruppen handelt es sich beispielsweise um leicht solvolytisch abspaltbare Acylgruppen oder hydrierend abspaltbare Gruppen. Die solvolytisch abspaltbaren Schutzgruppen werden beispielsweise durch Verseifung mit verdünnten Säuren (zum Beispiel Essigsäure, Perchlorsäure, Salzsäure, Schwefelsäure, Ameisensäure, Trifluoressigsäure) oder mittels basischer Substanzen (Pottasche, Soda, wässrige Alkalilösungen, alkoholische Alkalilösungen, $NH_3$) bei Temperaturen zwischen -50 und 150° C, insbesondere zwischen 0 und 100° C, abgespalten. Hydrierend abspaltbare Gruppen wie Arylalkylreste (Benzylrest) oder Aralkylcarbonylreste (Carbobenzoxyrest) werden zweckmäßig durch katalytische Hydrierung in Gegenwart üblicher Hydrierungskatalysatoren (Edelmetallkatalysatoren), insbesondere Palladium-Katalysatoren oder auch Platinkatalysatoren (Platinoxyd) oder Raney-Nickel, in einem Lösungs-oder Suspensionsmittel, gegebenenfalls unter erhöhtem Druck (züm Beispiel 1 - 50 bar) bei Temperaturen zwischen 20 - 150° C, insbesondere 30 - 100° C, vorzugsweise 40 - 80° C abgespalten. Als Lösungs- beziehungsweise Suspensionsmittel für die Abspaltung solcher Schutzgruppen kommen beispielsweise in Betracht: Wasser, niedere aliphatische Alkohole, cyclische Ether wie Dioxan oder Tetrahydrofuran, aliphatische Ether, Halogenkohlenwasserstoffe, Dimethylformamid und so weiter sowie Mischungen dieser Mittel. Als Schutzgruppen, die durch Hydrogenolyse abspaltbar sind, kommen beispielsweise in Frage: Benzylrest, α-Phenyläthylrest, im Benzolkern substituierte Benzylreste (p-Brom-oder p-Nitrobenzylrest), Carbobenzoxyrest beziehungsweise Carbobenzthiorest (wobei in solchen Resten der Benzolkern auch substituiert sein kann, beispielsweise durch $NO_2$), tert.-Butyloxycarbonylrest. Beispiele für hydrolytische abspaltbare Reste sind: Phthalylrest, Tritylrest, p-Toluolsulfonylrest, tert.-Butyloxycarbonylrest, tert.-Butylrest, Dimethylmethylenrest und ähnliche sowie niedere Alkanoylreste wie Acetylrest, Propionylrest, Trifluoracetylrest, Formylrest und ähnliche.

Falls die Ausgangsstoffe freie Carboxygruppen enthalten, ist es häufig zweckmäßig, diese vorher zu verestern, beispielsweise mit Benzylalkohol oder einem anderen niederen aliphatischen Alkohol (1 - 6, insbesondere 1 - 3 C-Atome). In den Endprodukten können derartige Estergruppen mittels Basen, beispielsweise alkoholischer Alkalilauge (zum Beispiel methanolische KOH) oder gegebenenfalls auch mittels Mineralsäuren wie Salzsäure oder Schwefelsäure in alkoholischer oder wässrig alkoholischer Lösung bei Temperaturen zwischen 20 und 100° C oder durch Hydrogenolyse abgespalten werden. Insbesondere kommen die bei der Peptid-Synthese üblichen Schutzgruppen und die dort üblichen Abspaltungsverfahren in Frage. Unter anderem wird hierzu auch auf das Buch von Jesse P. Greenstein und Milton Winitz "Chemistry of Amino Acids", New York, 1961, John Wiley and Sons, Inc., Volume 2, beispielsweise Seite 883 und folgende verwiesen. Auch die Carbalkoxygruppe (zum Beispiel niedrigmolekulare) kommt in Frage.

Die erfindungsgemäßen Verbindungen haben ein chirales Zentrum am 7-Ring sowie in den meisten Fällen weitere asymmetrische Kohlenstoffatome im Aminosäureteil A. Die Verbindungen der Formel I werden daher in der Regel als Racemate beziehungsweise Diastereomere erhalten. Solche Racemate können in an sich bekannter Weise beispielsweise durch fraktionierte Kristallisation der Salze von racemischen Verbindungen I mit optisch aktiven Säuren oder optisch aktiven Basen oder auch durch chromatographische Racemattrennung (siehe beispielsweise Angewandte Chemie 92/1 (1980) Seite 14) in die optisch aktiven Isomere beziehungsweise Enantiomere gespalten werden. Es ist aber auch möglich, von vornherein eine optisch aktive Ausgangssubstanz einzusetzen, wobei dann als Endprodukte eine entsprechende optisch aktive Form erhalten wird. Bei weiteren asymmetrischen C-Atomen erhält man diastereomere Gemische. Trennung kann nach den hierfür bekannten Methoden erfolgen.

Die vorliegende Erfindung umfasst also die Racemate und diastereomeren Formen sowie die entsprechenden optisch aktiven rechts-und linksdrehenden Formen.

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali, Säuren oder Ionenaustauschern wieder in die Basen übergeführt werden. Von den letzteren lassen sich durch Umsetzüng mit organischen oder anorganischen Säuren oder mit basischen Verbindungen insbesondere solchen, die zur Bildung von therapeutisch verwendbaren Salzen geeignet sind, Salze gewinnen.

Als Säuren für solche Salze kommen beispielsweise in Frage: Halogenwasserstoffsäuren, Schwefelsäure, Phosphorsäuren, Salpetersäure, Perchlorsäure, organische Mono-, Di-oder Tricarbonsäuren der aliphatischen, alicyclischen, aromatischen oder heterocyclischen Reihe sowie Sulfonsäuren. Beispiele hierfür sind: Ameisen-, Essig-, Propion-, Bernstein-, Glykol-, Milch-, Äpfel-, Wein-, Zitronen-, Ascorbin-, Malein-, Fumar-, Hydroxymalein-, Glucon-oder Brenztraubensäure; Phenylessig-, Benzoe-, p-Aminosalicylsäure, Embonsäure, Methansulfon-, Ethansulfon-, Hydroxyethansulfon-, Ethylensulfonsäure; Halogenbenzolsulfon-, Toluolsulfon-, Naphthalinsulfonsäure oder Sulfanilsäure oder auch 8-Chlor-theophyllin.

Die erfindungsgemässen Verbindungen sind zur Herstellung pharmazeutischer Zusammensetzungen geeignet. Die pharmazeutischen Zusammensetzungen beziehungsweise Medikamente können eine oder mehrere der erfindungsgemässen Verbindungen enthalten. Zur Herstellung der pharmazeutischen Zubereitungen können die üblichen Träger-und Hilfsstoffe verwendet werden.

Zu dem Verfahren:

Das Verfahren wird bei Temperaturen zwischen 0 und 150°C vorzugsweise 40 und 100°C in inerten Lösungsmitteln oder Suspensionsmitteln durchgeführt. Als Lösungs-oder Dispergiermittel kommen beispielsweise in Betracht: gegebenenfalls durch Chlor oder Brom substituierte aromatische Kohlenwasserstoffe wie zum Beispiel Benzol, Toluol, Xylol, Chlorbenzol, Pyridin; niedrigmolekulare aliphatische Ketone (zum Beispiel 3 - 6 C-Atome) wie zum Beispiel Aceton, Methylethylketon; halogenierte Kohlenwasserstoffe wie zum Beispiel Chloroform, Tetrachlorkohlenstoff, Chlorbenzol, Methylenchlorid; niedrigmolekulare aliphatische Ether (zum Beispiel 4 - 10 C-Atome) wie zum Beispiel Dimethoxyethan, Butylether; gesättigte cyclische Ether wie zum Beispiel Tetrahydrofuran, Dioxan; Sulfoxide wie zum Beispiel Dimethylsulfoxid; tertiäre Säureamide wie zum Beispiel Dimethylformamid, Tetramethylharnstoff, N-Methylpyrrolidin, Hexamethylphosphorsäuretriamid; Acetonitril; niedrigmolekulare aliphatische Alkohole wie Methanol, Ethanol, Isopropanol, Amylalkohol, tert.-Butanol; cycloaliphatische Kohlenwasserstoffe wie Cyclohexan; niedrigmolekulare gesättigte Chlor-und Fluorkohlenwasserstoffe mit 1 - 5 C-Atomen, wobei die einzelnen C-Atome ein-oder mehrfach (2-bis 3-fach) durch Chlor und/oder Fluor substituiert sein können, wie Chloroform, Methylenchlorid. Auch wässrige Mischungen der genannten Lösungsmittel können verwendet werden. Häufig arbeitet man bei der Rückflußtemperatur der verwendeten Lösungs-beziehungsweise Dispergiermittel.

Gegebenenfalls kann man auch so vorgehen, daß man zuerst von der Verbindung II eine Alkaliverbindung (Natrium-, Kalium-oder auch Lithiumsalz zum Beispiel) herstellt, indem man sie in einem inerten Lösungsmittel wie Dioxan, Dimethylformamid, Benzol oder Toluol mit einem Alkalimetall, Alkalihydrid oder Alkaliamiden (insbesondere Natrium oder Natriumverbindungen) oder Butyllithium bei Temperaturen zwischen 0 und 150° C reagieren läßt, und dann mit der Verbindung III (zum Beispiel in Form des Säurehalogenids) umsetzt.

Falls die freie Säure der Formel III verwendet wird, so ist deren Aktivierung durch die Gegenwart von Kondensationsmitteln wie Dicyclohexylcarbodiimid, Schwefligsäure-bis-alkylamiden (züm Beispiel $SO[N(CH_3)_2]_2$), N,N′-Carbonyldiimidazol und so weiter zweckmäßig (Organic Reactions, Vol. 12, 1962, Seiten 205 und 239).

Wird bei dem erfindungsgemäßen Verfahren eine Säure der Formel III mit aktivierter Carboxylgruppe eingesetzt, dann handelt es sich vorzugsweise um Verbindungen der allgemeinen Formel

$$R_3 - \underset{\displaystyle NR_4R_5}{\underset{|}{CH}} - COW \qquad IV$$

worin $R_3$, $R_4$ und $R_5$ die angegebenen Bedeutungen haben, und W ein Halogenatom, eine Gruppe der Formel $-OR'$, $-SR'$ oder eine Gruppe der Formel $-OCO-R''$ bedeutet und $R'$ einen $C_1$-$C_6$-Alkylrest oder im Falle von $-OR'$ beziehungsweise $-SR'$ auch einen Phenylrest, einen durch Nitrogruppen, $C_1$-$C_4$-Alkoxygruppen, $C_1$-$C_4$-Alkyl-gruppen oder Halogenatome (Chlor, Fluor, Brom) substituierten Phenylrest, einen Cyanmethylrest oder einen Carboxymethylrest und $R''$ einen geraden oder verzweigten $C_1$-$C_6$-Alkylrest, einen $C_1$-$C_6$-Alkoxyrest, einen Phenoxyrest oder einen Carbobenzoxyrest oder auch den Rest $R_3$-$CH(NR_4R_5)$ darstellt.

Falls W ein Halogenatom bedeutet, handelt es sich vorzugsweise um Chlor, Brom oder Jod; falls $R'$ beziehungsweise $R''$ Alkylreste oder Alkoxyreste bedeuten, dann sind diese vorzugsweise niedermolekular und bestehen aus 1-4 Kohlenstoffatomen.

Häufig, insbesondere wenn W (Formel IV) ein Halogenatom oder die Gruppe -OCOR bedeutet, ist die Gegenwart von säurebindenden Stoffen wie Alkalihydroxiden, Alkalicarbonaten, Alkalihydrogencarbonaten, Alkaliacetaten, Erdalkalicarbonaten, Trialkylaminen, Dialkylaminen, Pyridin und ähnlichen oder auch überschüssige Verbindung II zweckmäßig. Dabei kann das säurebindende Agens auch gleichzeitig allein oder im Gemisch mit anderen üblichen Mitteln als Lösungsmittel benutzt werden (zum Beispiel Pyridin). Es ist darauf zu achten, daß nichtumgesetzte Ausgangssubstanz III beziehungsweise IV, insbesondere falls es sich um ein Säurechlorid handelt, sorgfältig neutralisiert und entfernt wird. Häufig empfiehlt sich eine chromatographische Reinigung des Reaktionsproduktes über Kieselgel, wobei zum Beispiel mit einer Chloroform-Ethanol-Mischung (Ethanolgehalt zum Beispiel 4 %) gegebenenfalls unter Zusatz von wässrigem Ammoniak eluiert wird.

Anstelle der angeführten Aktivierungsmittel für die Carboxylgruppe können auch andere in der Chemie gebräuchliche chemisch-äquivalente Mittel verwendet werden. Derartige Mittel und Verfahren sind zum

Beispiel angegeben in folgenden Literaturstellen: L.F. und Mary Fieser "Reagents for Organic Synthesis", John Wiley and sons, Inc., New York, 1967, Vol. 1, Seiten 1303-4 and Vol. 2, Seite 471); Jakubke, Jeschkeit, "Aminosäuren, Peptide, Proteine", Akademie-Verlag, Berlin, 1982, 2. Auflage, Seiten 158 - 183. Die zuvor genannten Literaturstellen und deren Inhalt gemäß den angegebenen Seitenzahlen sind Bestandteil der Anmeldung.

Nicht bekannte Ausgangsverbindungen der Formel II können analog der deutschen Patentschrift 21 64 058 beziehungsweise der deutschen Offenlegungsschrift 35 30 793 erhalten werden. Die entsprechenden als Ausgangssubstanzen verwendeten 2-Phenacetyl-benzoesäuren enthalten im Phenacetylrest den Rest $R_1$ und gegebenenfalls $R_2$. Im allgemeinen stellt man zweckmäßig zuerst die entsprechende Verbindung II her, die am Stickstoff des 7-Ringes eine Methylgruppe enthält, und spaltet diese über die Carbethoxy-Verbindung analog den Beispielen der deutschen Offenlegungsschrift 35 30 793 ab. Die zugrund liegenden Verbindungen II mit der Methylgruppe am Stickstoff des 7-Ringes können ebenfalls gemäß beziehungsweise analog der deutschen Offenlegungsschrift 21 64 058 hergestellt werden.

Weitere pharmakologische und pharmazeutische Angaben

Die erfindungsgemäßen Verbindungen zeigen im Asthmaversuch an wachen Meerschweinchen eine gute antiasthmatische und antiallergische Wirkung. Beispielsweise wird bei oben genannter Versuchsmethode bei einer Dosis von 3 mg/kg Körpergewicht beim Meerschweinchen eine asthmaprotektive Wirkung gegen das allergische Asthma erzielt.

Die niedrigste bereits wirksame Dosis in dem oben genannten Tierversuch ist beispielsweise

0,3 mg/kg oral

0,1 mg/kg intravenös

Als allgemeiner Dosisbereich für die Wirkung (Tierversuch wie oben) kommt beispielsweise in Frage:

0,1-30 mg/kg oral, insbesondere 1,0-10,0 mg/kg

0,1-10 mg/kg intravenös, insbesondere 0,3-5,0 mg/kg

Die Wirkungsrichtung der erfindungsgemäßen Verbindungen ist mit der Wirkung des bekannten Arzneimittelwirkstoffs Dinatriumcromoglicinsäure vergleichbar, jedoch bestehen hierzu insbesondere folgende Unterschiede: perorale Wirksamkeit, stärkere Wirksamkeit, lange Wirkungsdauer. Indikationen für die die erfindungsgemäßen aerbindungen in Betracht kommen können: Allergische Rhinitis, Asthma bronchiale.

Die pharmazeutischen Zubereitungen enthalten im allgemeinen zwischen 0,1 bis 30 vorzugsweise 0,3 bis 10 mg der erfindungsgemäßen aktiven Komponente.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 1 und 10 mg oder Lösungen, die zwischen 1 bis 10 Gewichtsprozent an aktiver Substanz enthalten.

Die Einzeldosis der erfindungsgemäßen aktiven Komponenten kann beispielsweise liegen

a) bei oralen Arzneiformen zwischen 0,1 - 30 mg, vorzugsweise 1 - 10 mg

b) bei parenteralen Arzneiformen (zum Beispiel intravenös, intramuskulär) zwischen 0,1 - 10 mg vorzugsweise 0,3 - 5,0 mg.

c) bei Arzneiformen zur Inhalation (Lösungen oder Aerosole) zwischen 1 - 10 mg vorzugsweise 3 - 5 mg

-(Die Dosen sind jeweils bezogen auf die freie Base)-

d) bei Arzneiformen zur rektalen oder vaginalen Applikation zwischen 0,5 - 50 mg vorzugsweise 1 - 30 mg

e) bei Arzneiformen zur lokalen Applikation auf die Haut und Schleimhäute (zum Beispiel in Form von Lösungen, Lotionen, Emulsionen, Salben und so weiter) zwischen 1 - 10 mg vorzugsweise 3 - 5 mg

Beispielsweise können 3mal täglich 1 bis 2 Tabletten mit einem Gehalt von 1 bis 10 mg wirksamer Substanz oder zum Beispiel bei intravenöser Injektion 1 bis 2mal täglich eine Ampulle von 1 bis 10 ml Inhalt mit 0,3 bis 5,0 mg Substanz empfohlen werden. Bei oraler Verabreichung ist die minimale tägliche Dosis beispielsweise 1 mg; die maximale tägliche Dosis bei oraler Verabreichung soll nicht über 30 mg liegen.

Für die Behandlung von Hunden und Katzen liege die orale Einzeldosis im allgemeinen zwischen ungefähr 0,5 und 30 mg/kg Körpergewicht; die parenterale Dosis ungefähr zwischen 0,1 und 10 mg/kg Körpergewicht.

Für die Behandlung von Pferden und Vieh liegt die orale Einzeldosis im allgemeinen zwischen ungefähr 0,5 und 30 mg/kg; die parenterale Einzeldosis ungefähr zwischen 0,1 und 10 mg/kg Körpergewicht.

-(Die Dosen sind jeweils bezogen auf die freie Base)-

Die akute Toxizität der erfindungsgemäßen Verbindungen an der Maus (ausgedrückt durch die LD 50

mg/kg; Methode nach Miller und Tainter: Proc. Soc. Exper. Biol. a. Med. 57 (1944) 261) liegt beispielsweise bei oraler Applikation zwischen 250 und 1000 mg/kg.

Die Arzneimittel können in der Humanmedizin, der Veterinärmedizin sowie in der Landwirtschaft allein oder im Gemisch mit anderen pharmakologisch aktiven Stoffen verwendet werden.

Beispiel 1

4-(p-Chlor-benzyl)-2-[hexahydro-1-glycyl-azepin-4-yl]-1-(2H)-phthalazinon

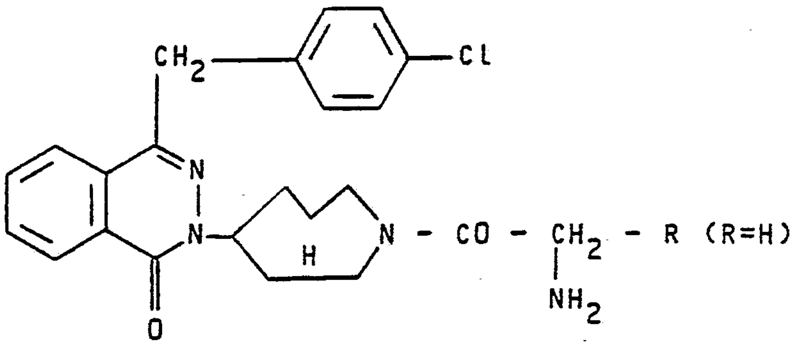

Zu einer Lösung von 5,26 g (0,03 Mol) N-tert.-Butyloxycarbonylglycerin in 50 ml Dioxan werden unter Rühren bei Raumtemperatur 5 g (0,031 Mol) 1,1-Carbonyldiimidazol zugegeben. Nach 60 Minuten Rühren wird bei Raumtemperatur eine Lösung von 11 g (0,03 Mol) 4-(p-Chlor-benzyl)-2-[hexahydroazepin-4-yl]-1-(2H)-phthalazinon (=Desmethylazelastin), in 80 ml Dioxan gelöst, zugetropft und die Mischung 4 Stunden bei 40 - 50° C gerrührt. Nach dem Dünnschichtchromatogramm enthält die Reaktionsmischung noch etwa 20 % nicht umgesetztes Desmethylazelastin. Es werden daher nochmals 1,1 g N-tert.-Butyloxycarbonylglycerin in 20 ml Dioxan gelöst, mit 1 g Carbonyldiimidazol versetzt und nach 10 Minuten unter Rühren der Reaktionsmischung zugefügt und das ganze nochmals einige Stunden (zum Beispiel 2 Stunden) bei 40° C gerührt. Danach wird das Lösungsmittel am Rotationsverdampfer entfernt, der Rückstand mit Wasser versetzt und 3mal mit Ether extrahiert. Die Etherlösung wird dann 2mal mit Wasser gewaschen, mit $MgSO_4$ getrocknet und eingeengt. Der ölige Rückstand wird in 50 ml Isopropanol gelöst und die Lösung mit isopropanolischer Salzsäure (5 - 10 ml) angesäuert und etwa 4 Stunden auf dem Wasserbad erhitzt (zur Abspaltung der Butyloxycarbonyl-Schutzgruppe).

Nun wird mit Eis gekühlt, wobei sich das Reaktionsprodukt als Öl abscheidet. Der ölige Rückstand wird mit Diethylether versetzt, man reibt kräftig an und saugt den Rückstand ab, versetzt mit isopropanolischer Salzsäure, engt ein, versetzt den Rückstand mit 50 ml Aceton und engt wieder am Rotationsverdampfer ein. Der nun erhaltene feste Rückstand wird aus 25 ml Isopropanol und Aktivkohle umkristallisiert, mit Ether gewaschen und bei 50° C in der Trockenpistole getrocknet.

Schmelzpunkt des Hydrochlorids: 187° C. Ausbeute: 2,3 g. Analog dem vorangegangenen Beispiel werden die Verbindungen gemäß der Tabelle 1 hergestellt. Es handelt sich hierbei um Verbindungen der bei Beispiel 1 angegebenen allgemeinen Formel.

Die Herstellung und Aufarbeitung erfolgt analog der bei Beispiel 1 angegebenen Weise.

Es werden jeweils 0,03 Mol der geschützten Aminosäure (zum Beispiel N-tert.-Butyloxycarbonyl-aminosäure) mit 0,025 - 0,03 Mol 4-(p-Chlor-benzyl)-2-hexahydroazepin-4-yl -1-(2H)-phthalazinon (=Desmethylazelastin) in einem Lösungsmittel (Dichlormethan, Dioxan) umgesetzt. Falls das Lösungsmittel Dichlormethan ist, erfolgt die Reaktion unter Rückflußtemperatur. In diesem Falle wird nach Beendigung direkt mit Wasser versetzt (zum Beispiel 100 ml), die organische Phase abgetrennt mit Wasser gewaschen und das Lösungsmittel entfernt.

Die Abspaltung der Aminosäureschutzgruppe erfolgt beispielsweise durch Erhitzen mit Salzesäure in Isopropanol; Dauer 1 - 4 Stunden.

Bei Beispiel 2 wird das nach Abspalten der Schutzgruppe erhaltene Produkt (Hydrochlorid) mit Wasser versetzt zur Entfernung von Verunreinigungen mit Diethylether ausgeschüttelt und anschließend das salzsaure Salz aus der wässrigen Lösung mit Dichlormethan extrahiert. Der nach Entfernen des Dichlormethan erhaltene Rückstand wird durch kurzes Erhitzen mit Diisopropyleter, dann durch Kristallisation zuerst aus Isopropanol + Kohle, dann aus Methanol + Kohle gereinigt.

Bei Beispiel 3 wird das nach Abspaltung der Schutzgruppe erhaltene Rohprodukt in 50 ml Wasser gelöst, 2mal mit tert.-Butylmethylether ausgeschüttelt, in der wässrigen Lösung mit $NH_3$ die Base freigesetzt und diese mit Dichlormethan extrahiert. Die isolierte Base wird in 50 ml Aceton gelöst und mit 2,8 ml isopropanolischer Salzsäure (5N HCl) in das Hydrochlorid überführt. Das Aceton wird entfernt, der Rückstand in abolutem Ethanol gelöst, das Ethanol entfernt und das so erhaltene Hydrochlorid mit 100 ml Isopropylether zum Sieden erhitzt und vom Isopropylether abdekantiert und getrocknet.

Bei Beispiel 4 wird nach Abspaltung der Schutzgruppe die salzsaure Isopronol-Reaktionslösung mit 100 ml Diethylether versetzt und auf - 20° C abgekühlt. Das Reaktionsprodukt scheidet sich als Öl ab. Nach Behandeln mit Aktivkohle in Isopropanol und Ethanol und Entfernen des Lösungsmittels wird der Rückstand mit Wasser versetzt, mit $NH_3$ die Base freigesetzt, die Base mit Ether extrahiert, nach Entfernen des Ethers die Base in 50 ml Aceton gelöst und mit 2,4 ml salzsaurem Isopropanol das Hydrochlorid hergestellt. Das Isopropanol wird entfernt und der Rückstand in 100 ml Diisopropylether zum Sieden erhitzt und nach Abkühlen das salzsaure Salz abgesaugt und bei 60° C im Vakuum getrocknet.

Tabelle 1

| Beispiel-Nr. | R | Schmelzpunkt des HCl-Salzes; °C | Aminosäure-Ausgangsverbindung | Reaktionsmedium | Ausbeute |
|---|---|---|---|---|---|
| 2 | $-CH_3$ | 163 | BOC-L-Alanin | $CH_2Cl_2$ (110 ml insgesamt) | 7,45 g |
| 3 | $-CH(CH_3)_2$ | 146 | BOC-L-Valin | $CH_2Cl_2$ (115 ml insgesamt) | 5,7 g |
| 4 | $-CH_2-C_6H_5$ | 154 | BOC-L-Phenylalanin | $CH_2Cl_2$ (100 ml insgesamt) | 5,4 g |

BOC = tert.-Butyloxycarbonyl

Beispiele für pharmazeutische Zubereitungen

<u>Tabletten</u> mit 4 mg Verbindung gemäß Beispiel 1

40 g Wirkstoff gemäß Beispiel 1, 460 g Lactose, 150 g Maisstärke und 10 g Aerosil werden durch ein Sieb (Maschenweite 0,8 mm) gegeben, gemischt und mit 200 g einer 5%igen wässrigen Gelatinelösung in der Wirbelschicht granuliert. Das trockene Granulat, 242 g mikrokristalline Cellulose, 80 g Maisstärke und 8 g Magnesiumstearat werden gesiebt (0,8 mm Maschenweite), homogen gemischt und in üblicher Weise zu Tabletten von 100 mg Gewicht und einem Durchmesser von 6 mm gepreßt.

1 Tablette enthält 4 mg Wirkstoff.

<u>Kapseln</u> mit 8 mg Verbindung gemäß Beispiel 2

80 g Wirkstoff nach Beispiel 2, 50 g Maisstärke, 1040 g Calciumhydrogenphosphatdihydrat und 10 g hochreines Siliciumdioxid (Aerosil) werden durch ein Sieb (0,8 mm Maschenweite) gegeben, homogenisiert und in der Wirbelschicht mit 200 g einer 10%igen wässrigen Gelatinelösung granuliert. 200 g Maisstärke und das trockene Granulat werden gesiebt (0,8 mm Maschenweite), homogenisiert und in üblicher Weise zu 140 mg in Hartgelatinekapseln der Größe 3 dosiert. 1 Kapsel enthält 8 mg Wirkstoff.

**Ansprüche**

1. Verbindungen der Formel

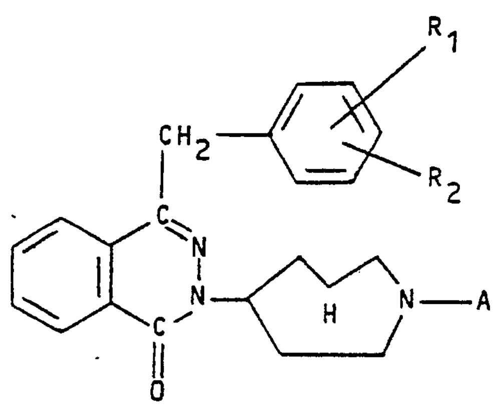

I

worin $R_1$ und $R_2$ Wasserstoff, Fluor, Chlor, Brom, Trifluormethyl-, $C_1$-$C_6$-Alkyl oder $C_1$-$C_6$-Alkoxy bedeuten, und der Rest A die Gruppe

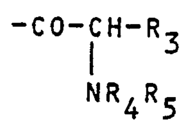

darstellt, worin $R_3$ Wasserstoff, einen Phenylrest, einen Indolyl-(3)-methylrest, Imidazolyl-(4)-methylrest, eine $C_1$-$C_{10}$-Alkylgruppe bedeutet oder worin $R_3$ eine $C_1$-$C_{10}$-Alkylgruppe bedeutet, welche durch eine Carboxygruppe, eine $C_1$-$C_6$-Alkoxycarbonylgruppe eine Aminocarbonylgruppe, eine Hydroxygruppe, eine

$C_1$-$C_6$-Alkoxygruppe, eine $C_2$-$C_6$-Alkanoyloxygruppe, eine Mercaptogruppe, eine $C_1$-$C_6$-Alkylthiogruppe, eine $C_2$-$C_6$-Alkanoylmercaptogruppe, eine Phenylgruppe, eine Hydroxyphenylgruppe, eine Dihydroxyphenylgruppe, eine Amino-$C_1$-$C_6$-alkylthiogruppe, eine Amino-$C_1$-$C_6$-alkyloxygruppe, eine Aminogruppe, eine Ureidogruppe ($H_2NCONH$-) oder eine Guanidinogruppe substituiert ist, oder worin $R_3$ zusammen mit dem Strukturteil $>CH(NHR_4)$ den Pyrrolidin-2-yl-rest (Prolinrest) oder den 4-Hydroxy-pyrrolidin-2-yl-rest darstellt, $R_4$ Wasserstoff, Benzyl oder einen $C_1$-$C_6$-Alkylrest, $R_5$ Wasserstoff, Benzyl, einen $C_1$-$C_6$-Alkylrest, einen $C_2$-$C_6$-Alkanoylrest oder die Gruppe

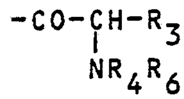

bedeutet, wobei $R_3$ und $R_4$ die bereits angegebenen Bedeutungen haben und $R_6$ Wasserstoff, Benzyl oder $C_2$-$C_6$-Alkanoyl ist, und deren physiologisch verträglichen Salze.

2. Verfahren zur Herstellung von Verbindungen der Formel

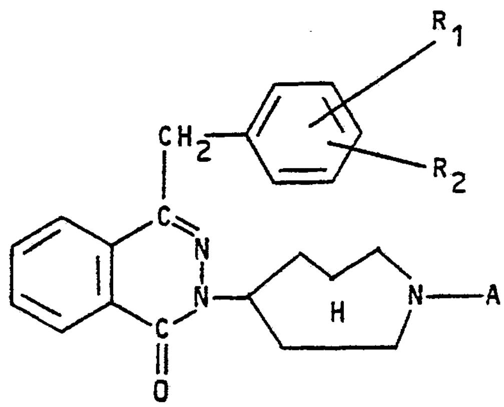

I

worin $R_1$ und $R_2$ Wasserstoff, Fluor, Chlor, Brom, Trifluormethyl, $C_1$-$C_6$-Alkyl oder $C_1$-$C_6$-Alkoxy bedeuten, und der Rest A die Gruppe

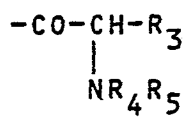

darstellt, worin $R_3$ Wasserstoff, einen Phenylrest, einen Indolyl-(3)-methylrest, Imidazolyl-(4)-methylrest, eine $C_1$-$C_{10}$-Alkylgruppe bedeutet oder worin $R_3$ eine $C_1$-$C_{10}$-Alkylgruppe bedeutet, welche durch eine Carboxygruppe, eine $C_1$-$C_6$-Alkoxycarbonylgruppe, eine Aminocarbonylgruppe, eine Hydroxygruppe, eine $C_1$-$C_6$-Alkoxygruppe, eine $C_2$-$C_6$-Alkanoyloxygruppe, eine Mercaptogruppe, eine $C_1$-$C_6$-Alkylthiogruppe, eine $C_2$-$C_6$-Alkanoylmercaptogruppe, eine Phenylgruppe, eine Hydroxyphenylgruppe, eine Dihydroxyphenylgruppe, eine Amino-$C_1$-$C_6$-alkylthiogruppe, eine Amino-$C_1$-$C_6$-alkyloxygruppe, eine Aminogruppe, eine Ureidogruppe ($H_2NCONH$-) oder eine Guanidinogruppe substituiert ist, oder worin $R_3$ zusammen mit dem Strukturteil $>CH(NHR_4)$ den Pyrrolidin-2-yl-rest (Prolinrest) oder den 4-Hydroxy-pyrrolidin-2-yl-rest darstellt, $R_4$ Wasserstoff, Benzyl oder einen $C_1$-$C_6$-Alkylrest, $R_5$ Wasserstoff, Benzyl einen $C_1$-$C_6$-Alkylrest, einen $C_2$-$C_6$-Alkanoylrest oder die Gruppe

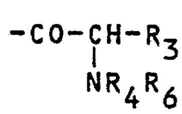

bedeutet, wobei $R_3$ und $R_4$ die bereits angegebenen Bedeutungen haben und $R_6$ Wasserstoff, Benzyl oder

$C_2$-$C_6$-Alkanoyl ist, und deren physiologisch verträglichen Salzen, dadurch gekennzeichnet, daß man eine Verbindung der allgemeinen Formel

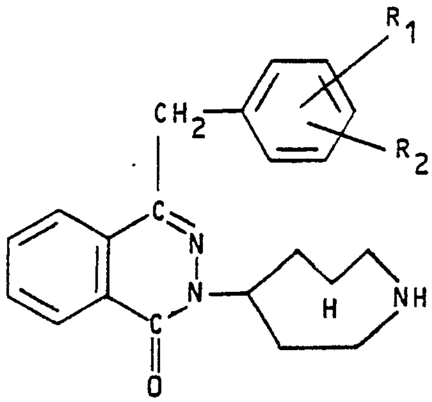

II

worin $R_1$ und $R_2$ die angegebenen Bedeutungen haben mit einer Säure der allgemeinen Formel

$$R_3 - \underset{\displaystyle NR_4R_5}{\underset{|}{CH}} - CO_2H \qquad III$$

worin $R_3$, $R_4$ und $R_5$ die angegebenen Bedeutungen haben, wobei die Carboxygruppe auch aktiviert sein kann, umsetzt, wobei vorhandene primäre oder sekundäre Aminogruppen, Carboxygruppen, Hydroxygruppen und/oder Mercaptogruppen der Ausgangsverbindung III durch übliche Schutzgruppen geschützt sein können, in den erhaltenen Verbindungen gegebenenfallsvorhandene Schutzgruppen abspaltet, gegebenenfalls vorhandene freie Hydroxygruppen, Mercaptogruppen, primäre oder sekundäre Aminogruppen alkyliert oder acyliert
und die erhaltenen Verbindungen gegebenenfalls in ihre Salze überführt.

3. Verbindungen der Formel I zur Anwendung als therapeutische Wirkstoffe.

4. Arzneimittel, enthaltend eine Verbindung der allgemeinen Formel I neben üblichen Träger-und/oder Verdünnungs-beziehungsweise Hilfsstoffen.

5. Verfahren zur Herstellung eines Arzneimittels, dadurch gekennzeichnet,
daß eine Verbindung der allgemeinen Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

6. Verwendung von Verbindungen der allgemeinen Formel I zur Herstellung Von Arzneimitteln.

Europäisches Patentamt

# EUROPÄISCHER RECHERCHENBERICHT

Nummer der Anmeldung

EP 88106802.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP - A2 - 0 174 464 (ASTA) * Ansprüche 1-6 * -- | 1-6 | C 07 D 403/04<br>C 07 D 403/14<br>A 61 K 31/50 |
| A | US - A - 3 813 384 (VOGELSANG) * Anspruch 1; Zusammenfassung * -- | 1,4 | |
| P,A | DE - A1 - 3 634 942 (ASTA) * Ansprüche 1-6 * -- | 1-6 | |
| A | CHEMICAL ABSTRACTS, Band 95, Nr. 21, 23. November 1981, Columbus, Ohio, USA<br>HASEGAWA, J.; TOMONO, Y.; TANAKA, M.; FUJITA, T.; SUGIYAMA, K.; HIROSE, N. "Pharmacokinetics and biopharmaceutical studies of azelastine hydrochloride in beagle dogs by quantitative selected-ion monitoring." Seite 7, Spalte 2, Zusammenfassung-Nr. 180 509r<br>& Arzneim.-Forsch. 1981, 31(8), 1215-20<br>---- | 1,4 | RECHERCHIERTE SACHGEBIETE (Int. Cl.4)<br>C 07 D 403/00 |

Der vorliegende Recherchenbericht wurde für alle Patentansprüche erstellt.

| Recherchenort | Abschlußdatum der Recherche | Prüfer |
|---|---|---|
| WIEN | 22-06-1988 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82